# EUROPÄISCHE PATENTSCHRIFT

(11) **EP 0 355 645 B1**
(45) Veröffentlichungstag und Bekanntmachung des Hinweises auf die Patenterteilung: **27.04.1994**
(21) Anmeldenummer: 89114978.3
(22) Anmeldetag: 12.08.1989
(51) Int. Cl.: C07D 401/12, C08K 5/3435

(54) **Neue cyclische Amidine und Verfahren zu ihrer Herstellung**
Cyclic amidines and process for their preparation
Amidines cycliques et procédé de leur préparation

(30) Priorität: 25.08.1988 DE 3828759
(43) Veröffentlichungstag der Anmeldung: 28.02.1990
(73) Patentinhaber: BAYER AG, 51368 Leverkusen (DE)
(72) Erfinder: Köhler, Burkhard, Dr., D-4150 Krefeld 11 (DE); Meier, Helmut-Martin, Dr., D-4030 Ratingen 6 (DE)

(56) Entgegenhaltungen:
- EP-A- 0 212 479
- DE-A- 2 029 299
- DE-A- 2 119 163
- CHEMICAL ABSTRACTS, Band 58, Seite 1962, Zusammenfassung Nr. 11356f, Columbus, Ohio, US; H. BAGANZ et al.: "2-Alkylaminomethyl-2-imidazoline and tetrahydro-2-pyrimidine"

## Beschreibung

Die Erfindung betrifft cyclische Amidine, deren exocyclischer Stickstoff durch einen 2,2,6,6-Tetraalkylpiperidinrest substituiert ist.

Tetraalkylpiperidine werden bekanntlich zur Stabilisierung von synthetischen Polymeren eingesetzt (z.B. US-PS 4 322 531, EP-OS 6536; Polymer Photochemistry 2 (1982) 3, 175 ff). Cyclische Amidine können als Fungizide eingesetzt werden. Sie können aus dem entsprechenden cyclischen O-Methyl-Lactimether und einem primären Amin hergestellt - z.B. Appl. 6413085, "Methoden der organ. Chemie, Houben-Weyl, Bd. XI/2, S. 57, 4. Auflage, Verlag G. Thieme, Stuttgart 1958).

Es wurde nun gefunden, daß 4-Amino-2,2,6,6-tetraalkylpiperidine mit cyclischen O-Alkyl-Lactimethern, deren Salzen oder Tetrahydroazepine mit einer anderen Abgangsgruppe zu neuen cyclischen Amidinen reagieren.

Gegenstand der Erfindung sind cyclische Amidine der Formel (I) 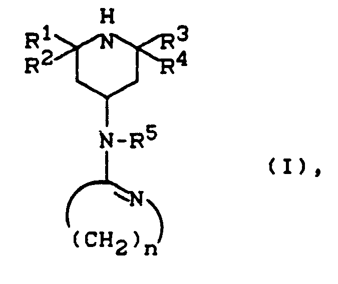
in welcher
- R¹ bis R⁴: gleich oder verschieden sind und C₁₋₂₂-Alkyl bedeuten,
- R⁵: für Wasserstoff, C₁₋₂₂-Alkyl, C₇₋₂₂-Aralkyl oder -Aryl-alkyl, vorzugsweise Benzyl, C₆₋₁₄-Aryl, vorzugsweise Phenyl, besonders bevorzugt Wasserstoff bedeutet und
- n: für die Zahl 3, 4, 5, 6, 7, 8, 9, 10, 11, bevorzugt 5 steht.

Als Reste R¹, R², R³ und R⁴ sind beispielsweise genannt: Methyl, Ethyl, Propyl, i-Propyl, Butyl, Hexyl, Cyclohexyl, Heptyl, Nonyl, Decyl, Undecyl usw. Bevorzugt ist Methyl.

Als Reste R⁵ sind beispielsweise die für R¹ bis R⁴ genannt, Benzyl, Naphthyl, Methylphenyl, Ethylphenyl u.s.w.

Ein weiterer Gegenstand der Erfindung ist ein Verfahren zur Herstellung der cyclischen Amidine der Formel (I) 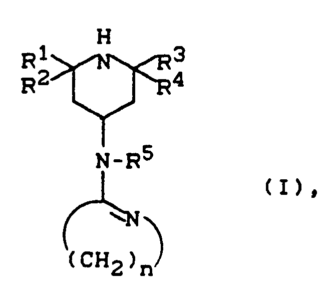
in welcher
- R¹ bis R⁴: gleich oder verschieden sind und C₁₋₂₂-Alkyl bedeuten,
- R⁵: für Wasserstoff, C₁₋₂₂-Alkyl, C₇₋₂₂-Aralkyl oder -Aryl-alkyl, vorzugsweise Benzyl oder C₆₋₁₄-Aryl, vorzugsweise Phenyl, besonders bevorzugt Wasserstoff bedeutet und
- n: für die Zahl 3, 4, 5, 6, 7, 8, 9, 10, 11, bevorzugt 5 steht,
dadurch gekennzeichnet, daß in an sich bekannter Weise 4-Amino-2,2,6,6-tetraalkylpiperidine der Formel (II) 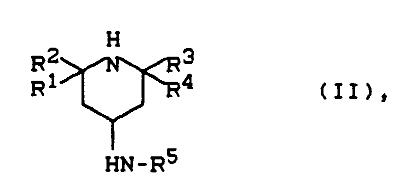
in welcher
- R¹ bis R⁵: die bereits angegebene Bedeutung haben,
mit Heterocyclen der Formel (III) 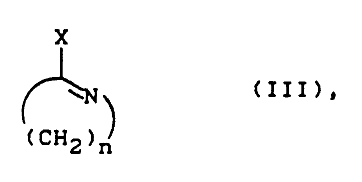
in welcher
- n: die bei Formel (I) angegebene Bedeutung hat
und
- X: für eine Gruppe der Formeln (IV), (V) oder (VI)

-S-R⁶ (IV),

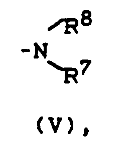

-O-R⁶ (VI),

steht,
in welchen
- R⁶: C₁₋₆-Alkyl, vorzugsweise Methyl oder Ethyl, C₇₋₂₂-Alkylaryl oder Arylalkyl, bevorzugt Benzyl oder C₆₋₂₂-Aryl vzw. Phenyl bedeutet und
- R⁷ und R⁸: gleich oder verschieden sind und Wasserstoff C₁₋₂2-Alkyl, bevorzugt Methyl, Ethyl, Propyl oder Butyl oder C₆₋₂₂-Aryl, bevorzugt Phenyl bedeuten,
in Gegenwart von Katalysatoren in einem Lösungsmittel umgesetzt werden.

Beispiele für erfindungsgemäß einsetzbare Verbindungen der Formel II sind 4-Amino-2,2,6,6-tetramethylpiperidin, 4-Methyl-amino-2,2,6,6-tetramethylpiperidin, 4-Phenylamino-2,2,6,6-tetramethylpiperidin, 4-Amino-2,2,6,6-tetraethylpiperidin, 4-Methylamino-2,2,6,6-tetraethylpiperidin, 4-Phenylamino-2,2,6,6-tetraethylpiperidin usw.

4-Amino-2,2,6,6-Tetramethylpiperidin kann beispielsweise nach Liebigs Ann. Chem. 417, 118 bis 119 (1918) aus dem entsprechenden Oxim durch Reduktion mit Zinkstaub hergestellt werden.

Beispiele für erfindungsgemäß einsetzbare Verbindungen der Formel III sind 2-Methoxy-Δ¹-1-tetrahydroazepin, 2-Ethoxy-Δ¹-1-tetrahydroazepin, 2-Methoxy-Δ¹-pyrrolin, 2-Ethoxy-Δ¹-1-pyrrolin, 2-Methylmercapto-Δ¹-1-tetrahydroazepin, 2-Ethylmercapto-Δ¹-1-tetrahydroazepin, 2-Phenylthio-Δ1-tetrahydroazepin, 2-Amino-Δ1Tetrahydroazepin, 2-Dimethylamino-Δ1-tetrahydroazepin, 2-Methoxy-1-azacyclotridecen-1 oder 2-Methylmercapto-1-azacyclotridecen-1 usw.

Die Methoxyverbindungen der Formel (III) können z.B. aus dem entsprechenden Lactam und Dimethylsulfat nach J. Am. Chem. Soc 70, 2115 bis 2116 (1948) bzw. Org. Synth. Coll. Vol IV (1963) 588 hergestellt werden. Für die Herstellung der erfindungsgemäßen Produkte können auch die primär anfallenden Schwefelsäuremonomethylestersalze der Methoxyheterocyclen der Formel (III) eingesetzt werden.

Erfindungsgemäß können als Katalysatoren Säuren, beispielsweise Protenensäuren wie Halogenwasserstoffsäuren, z.B. Salzsäure, Sulfonsäuren wie Fluorsulfonsäure, p-Toluolsulfonsäure, Borsäuren wie Tetrafluorborsäure, Schwefelsäure(ester), z.B. Schwefelsäuremonoester, Dimethylsulfat eingesetzt werden. Weiterhin können auch Basen als Katalysatoren eingesetzt werden, z.B. Amine wie Trialkylamine, z.B. Triethylamin, aromatische Amine, z.B. Dialkylaniline wie Dimethylanilin, heterocyclische Amine wie Pyridin, usw.

Pro Mol der Heterocyclen der Formel (II) werden 0,001 bis 1,5, vorzugsweise 0,01 bis 1 Äquivalente Katalysatoren eingesetzt.

Als Lösungsmittel können organische Lösungsmittel wie Alkohole (z.B. C₁-C₅-Alkohole wie Methanol, Ethanol), halogenierte Kohlenwasserstoffe, z.B. Chlorkohlenwasserstoffe wie Methylenchlorid, Chlorethane, Ether, Ester aromatische Lösungsmittel wie Toluol usw. eingesetzt werden.

Das erfindungsgemäße Verfahren wird bei Temperaturen von 0 bis 200°C durchgeführt, wobei katalysierte Reaktionen vorzugsweise bei Temperaturen von 0 bis 50°C und nicht katalysierte Reaktionen vorzugsweise bei Temperaturen von 100 bis 200°C durchgeführt werden.

Das erfindungsgemäße Verfahren wird bei Drücken von 0,1 bis 50 bar, vorzugsweise unter atmosphärischem Druck durchgeführt.

Das Verhältnis der Molmengen an Edukt der Formel (II) zum Edukt der Formel (III) beträgt 0,8:1 bis 1:1,2, vorzugsweise 1:1. Das Verhältnis Reaktionspartner zu Lösungsmittel beträgt 1:0 bis 1:20, vorzugsweise 1:3 bis 1:10.

Die Reaktionsdauer beträgt 10 min bis 10 h, wobei katalysierte Reaktionen zwischen 10 bis 60 min dauern.

Die Isolierung der Produkte der Formel (I) erfolgt durch Abdestillieren des Solvens und gegebenenfalls Entfernen von sauren Katalysatoren durch wäßrige Lösungen von Alkalihydroxiden, -carbonaten oder Hydrogencarbonaten. Die Produkte können gegebenenfalls durch Hochvakuumdestillation oder Umkristallisieren gereingt werden.

Die erfindungsgemäßen cyclischen Amidine eignen sich beispielsweise als Pflanzenschutzpräparate, Zwischenprodukte oder als Stabilisatoren für Kunststoffe, z.B. Polyolefine oder Polyvinylchlorid.

### Beispiel 1

Man versetzt 23,4 g 4-Amino-2,2,6,6-tetramethylpiperazin mit 19,05 g O-Methylcaprolactimether (2-Methyl-Δ1-tetrahydroazepin) und 0,3 g p-Toluolsulfonsäure. Dann wird 8 h auf 170°C erhitzt, wobei Methanol abdestilliert. Man nimmt in 150 ml Methylenchlorid auf und schüttelt mit gesättigter Natriumcarbonat aus. Die organische Phase wird eingeengt und der Rückstand aus Toluol umkristallisiert. Die Ausbeute an 2-(2′,2′,6′,6′-Tetramethylpiperidinyl-4′)-amino-Δ1-tetrahydroazepin beträgt 20,1 g, Fp 90°C.

### Beispiel 2

Man mischt 26 g Caprolactam und 25,2 g Dimethylsulfat und erhitzt 8 h auf 80°C. Dann werden 150 ml Methylenchlorid hinzugefügt und unter Eiskühlung die Lösung von 31,2 g 4-Amino-2,2,6,6-tetramethylpiperazin in 30 ml Methylenchlorid so zugetropft, daß die Temperatur unter 20°C bleibt. Man schüttelt mit 150 ml 10 Gew.-% NaOH aus, engt die organische Phase ein und kristallisiert aus Toluol um. Die Ausbeute an dem Produkt aus Beispiel 1 beträgt 42,7 g.

## Patentansprüche

1. Cyclische Amidine der Formel (I) 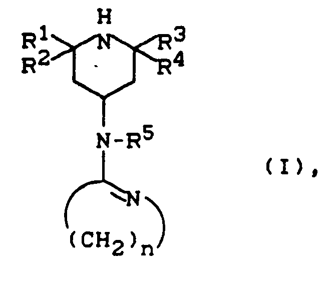 in welcher
R¹ bis R⁴ gleich oder verschieden sind und C₁₋₂₂-Alkyl
bedeuten,
R⁵ für Wasserstoff, C₁₋₂₂-Alkyl, C₇₋₂₂-Aralkyl oder -Aryl-alkyl, vorzugsweise Benzyl, C₆₋₁₄-Aryl, vorzugsweise Phenyl, besonders bevorzugt Wasserstoff bedeutet und
n für die Zahl 3, 4, 5, 6, 7, 8, 9, 10, 11, bevorzugt 5 steht.

2. Verfahren zur Herstellung der cyclischen Amidine der Formel (I) 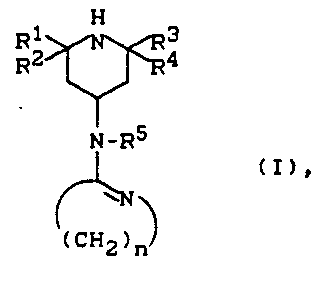 in welcher
R¹ bis R⁴ gleich oder verschieden sind und C₁₋₂₂-Alkyl bedeuten,
R⁵ für Wasserstoff, C₁₋₂₂-Alkyl, C₇₋₂₂-Aralkyl oder -Aryl-alkyl, vorzugsweise Benzyl oder
C₆₋₁₄-Aryl, vorzugsweise Phenyl,
besonders bevorzugt Wasserstoff bedeutet und
n für die Zahl 3, 4, 5, 6, 7 , 8, 9, 10, 11, bevorzugt 5 steht,
dadurch gekennzeichnet, daß in an sich bekannter Weise 4-Amino-2,2,6,6-tetraalkylpiperidine der Formel (II) 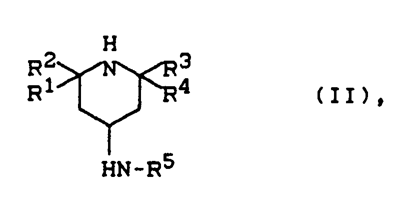 in welcher
R¹ bis R⁵ die bereits angegebene Bedeutung haben,
mit Heterocyclen der Formel (111) 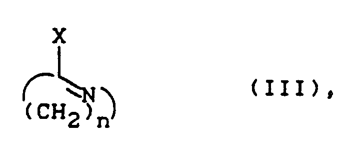 in welcher
n die bei Formel (I) angegebene Bedeutung hat
und
X für eine Gruppe der Formeln (IV), (V) oder (VI)
-S-R⁶ (IV), 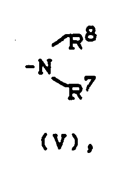
-O-R⁶ (VI),
steht,
in welchen
R⁶ C₁₋₆-Alkyl, vorzugsweise Methyl oder Ethyl, C₇₋₂₂-Alkylaryl oder Arylalkyl, bevorzugt Benzyl oder C₆₋₂₂-Aryl vzw. Phenyl bedeutet und
R⁷ und R⁸ gleich oder verschieden sind und Wasserstoff C₁₋₂2-Alkyl, bevorzugt Methyl, Ethyl, Propyl oder Butyl oder C₆₋₂₂-Aryl, bevorzugt Phenyl bedeuten,
in Gegenwart von Katalysatoren in einem Lösungsmittel umgesetzt werden.

3. Verwendung von cyclischen Amidinen nach Anspruch 1 als Zwischenprodukte zur Herstellung von Kunststoffen.

## Claims

1. Cyclic amidines corresponding to formula (I) 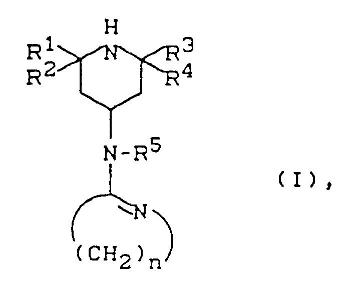 in which
R¹ to R⁴ may be the same or different and represent C₁₋₂₂ alkyl,
R⁵ represents hydrogen, C₁₋₂₂ alkyl, C₇₋₂₂ aralkyl or arylalkyl, preferably benzyl, C₆₋₁₄ aryl, preferably phenyl and, more preferably, hydrogen, and
n is the number 3, 4, 5, 6, 7, 8, 9, 10, 11, preferably 5.

2. A process for the production of the cyclic amidines corresponding to formula (I) 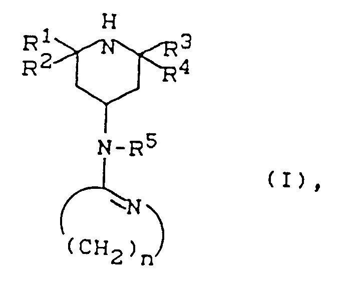 in which
R¹ to R⁴ may be the same or different and represent C₁₋₂₂ alkyl,
R⁵ represents hydrogen, C₁₋₂₂ alkyl, C₇₋₂₂ aralkyl or arylalkyl, preferably benzyl, C₆₋₁₄ aryl, preferably phenyl and, more preferably, hydrogen, and
n is the number 3, 4, 5, 6, 7, 8, 9, 10, 11, preferably 5,
characterized in that 4-amino-2,2,6,6-tetra-alkyl piperidines corresponding to formula (II) 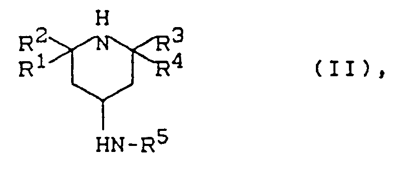 in which
R¹ to R⁵ are as already defined,
are reacted in known manner with heterocycles corresponding to formula (III) 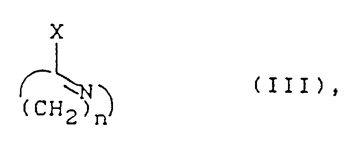 in which
n is as defined for formula (I) and
X represents a group corresponding to one of the following formulae:
-S-R⁶ (IV), 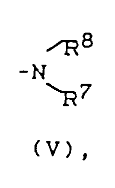
-O-R⁶ (VI),
in which
R⁶ represents C₁₋₆ alkyl, preferably methyl or ethyl, C₇₋₂₂ alkylaryl or arylalkyl, preferably benzyl, or C₆₋₂₂ aryl or phenyl and
R⁷ and R⁸ may be the same or different and represent hydrogen, C₁₋₂₂ alkyl, preferably methyl, ethyl, propyl or butyl or C₆₋₂₂ aryl, preferably phenyl,
in the presence of catalysts in a solvent.

3. The use of the cyclic amidines claimed in claim 1 as intermediate products for the production of plastics.

## Revendications

1. Amidines cycliques de formule (I) 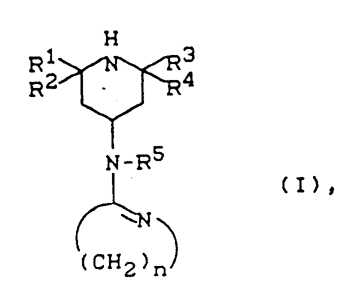 dans laquelle
R¹ à R⁴ sont identiques ou différents et représentent des groupes alkyle en C₁ à C₂₂,
R⁵ est de l'hydrogène, un groupe alkyle en C₁ à C₂₂, aralkyle ou aryl-alkyle en C₇ à C₂₂, de préférence benzyle, aryle en C₆ à C₁₄, de préférence phényle, notamment l'hydrogène et
n représente le nombre 3, 4, 5, 6, 7, 8, 9, 10 ou 11, de préférence 5.

2. Procédé de production des amidines cycliques de formule (I) 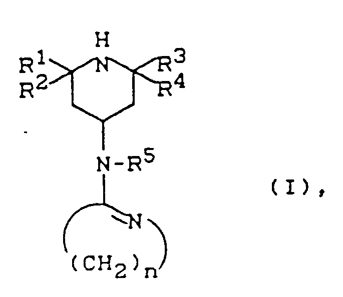 dans laquelle
R¹ à R⁴ sont identiques ou différents et représentent des groupes alkyle en C₁ à C₂₂,
R⁵ est de l'hydrogène, un groupe alkyle en C₁ à C₂₂, aralkyle ou aryl-alkyle en C₇ à C₂₂, de préférence benzyle, ou un groupe aryle en C₆ à C₁₄, de préférence phényle, notamment l'hydrogène et
n représente le nombre 3, 4, 5, 6, 7, 8, 9, 10 ou 11, de préférence 5,
caractérisé en ce qu'on fait réagir d'une manière connue des 4-amino-2,2,6,6-tétraalkylpipéridines de formule (II) 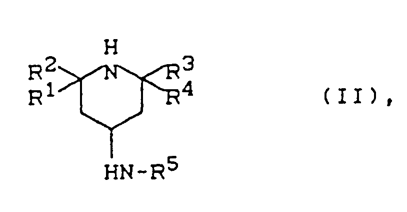 dans laquelle
R¹ à R⁵ ont la définition déjà indiquée,
avec des hétérocycles de formule (III) 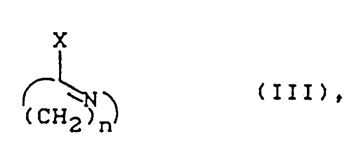 dans laquelle
n a la définition indiquée pour la formule (I)
et
X représente un groupe de formule (IV), (V) ou (VI)
-S-R⁶ (IV), 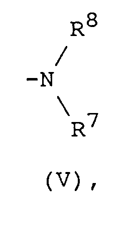
-O-R⁶ (VI),
où
R⁶ représente un groupe alkyle en C₁ à C₆, de préférence méthyle ou éthyle, un groupe alkylaryle ou arylalkyle en C₇ à C₂₂, de préférence benzyle ou un groupe aryle en C₆ à C₂₂, de préférence phényle et
R⁷ et R⁸ sont identiques ou différents et représentent de l'hydrogène, un groupe alkyle en C₁ à C₂₂, de préférence méthyle, éthyle, propyle ou butyle ou un groupe aryle en C₆ à C₂₂, de préférence phényle
en présence de catalyseurs dans un solvant.

3. Utilisation d'amidines cycliques suivant la revendication 1 comme produits intermédiaires pour la production de matières plastiques.
